# EUROPEAN PATENT APPLICATION

(11) **EP 2 524 962 A1**
(43) Date of publication of application: **21.11.2012**
(21) Application number: 12003850.0
(22) Date of filing: 16.05.2012
(51) Int. Cl.: C12M 1/00, C12N 1/12

(54) **Algae culture system**

(30) Priority: 17.05.2011 CL 11452011
(71) Applicant: Aeon Biogroup Spa, Santiago (CL)
(72) Inventor: Navarro, Alfonso, Las Condes, Santiage (CL)
(74) Representative: Brosch, Oliver

(57) **Abstract**

The invention relates to a microalgae culture system that provides a greater control of the culture due to distribution and the shape of its components, and the possibility to incorporate gases into the medium, resulting in an increased culture yield and lower energy consumption per volume unit. The invention comprises a pond (1) with a circular mantle, PVC parts and a removable lid. Each pond is provided with two aerators: one (12) placed over the liquid livel, adapted to introduce recirculated culture and air, and one shaped as a perforated semicicular pipe (16), placed in a lower position. The operation of both aerators achieves the movement of the culture liquid.

## Description

### SCOPE OF THE INVENTION

The invention relates to a microalgae culture system that delivers a greater control of the culture due to the distribution and shape of its components, and the possibility to incorporate gases into the medium, resulting in an increased culture yield and reduced energy consumption per volume unit.

### DESCRIPTION OF THE PRIOR ART

Microalgae have been used in aquaculture as a food supplement and in the production of chemical compounds (Raja et al, 2008), and more recently they have been proposed as an energy source for fuel production, offering several advantages over traditional cultures, such as high photosynthetic efficiency, high lipid content, continuous production of biomass and fast growth (Moo-Younga and Chisti, 1994; Sánchez et al, 2003; Miao and Wu, 2006), and also because they are a renewable source with low emissions of pollutants into the atmosphere (CO₂ and CO₂).

While the biological bases of the microalgae culture are widely developed on a small scale, they lack culturing capacity on a large scale to produce biomass at a low cost. For intensive production of microalgae, two culture systems are mainly used, open systems or *Raceway* ponds and closed systems or photobioreactors (PBRs). In open systems, cultures are exposed to the atmosphere in a type of channel of large dimensions and are constantly stirred by a *paddlewheel.* PBRs are highly productive culture systems that allow for a greater culture yield per area and volume unit, compared to open systems (Sánchez et al, 2003; Khan et al. 2009). PBRs can be made of plastic, glass and transparent PVC, among other materials, and in different shapes, horizontal, vertical, circular, etc.

The high productivity of PBRs is associated with the control of all culture parameters and the aseptic conditions they provide, which ultimately translate into higher productivity per volume unit. The production figures of both systems reach very different numbers. According to Sánchez, in terms of volume productivity in Kg/m³, photobioreactors are fifteen times more effective than open systems and use half the area measures in hectares. In addition to these advantages, we might add that open ponds can be contaminated and difficult to control in terms of culture conditions. However, installation costs are minimal compared to those incurred with PBRs, which at the same time are more difficult to clean. These are the main reasons that so far most of the microalgae production has been made in open systems.

Regarding the disadvantages of current systems using open ponds, the only production systems currently in use are open pools, of the *raceway* or circular type. These systems use a mechanical stirrer, of the paddlewheel type, which is in contact with the water and undergoes corrosion and wear. The movement resulting from this type of propulsion tends to be a laminar flow, which means an incorrect turbulence for the algae nutrition.

These systems were not designed to incorporate gases such as air or pure CO₂ into the medium, and the only source of CO₂ is a passive transfer of gas from the air into the water through the exposed surface. This creates a limitation in the capture of CO₂, resulting in low growth rates.

Escalation of these systems to sizes larger than current ones (100 ha) is impractical due to low productivity per area unit and the enormous loss of water by evaporation. These systems also tend to be contaminated with chemical and biological agents, such as larvae, bacteria, microalgae competing and predating species, which have presented serious problems for existing plants, significantly reducing the plant factor.

Finally, the use of moving parts or engines near or in contact with water increases the probability of failure, especially when using seawater.

### SUMMARY OF THE INVENTION

The system of the invention is a closed microalgae culture system that provides a greater control of the culture and the possibility to incorporate gases into the medium, resulting in an increased culture yield and lower energy consumption per volume unit. The invention uses a cellular model for ease of escalation, allowing the independence of the units and an escalation *ad infinitum.* The model physically consists of arrays of circular ponds with transparent lids, *airlift* water pumping systems connected to a loading/unloading system, provision of gases and an automatic control system.

### BRIEF DESCRIPTION OF THE DRAWINGS

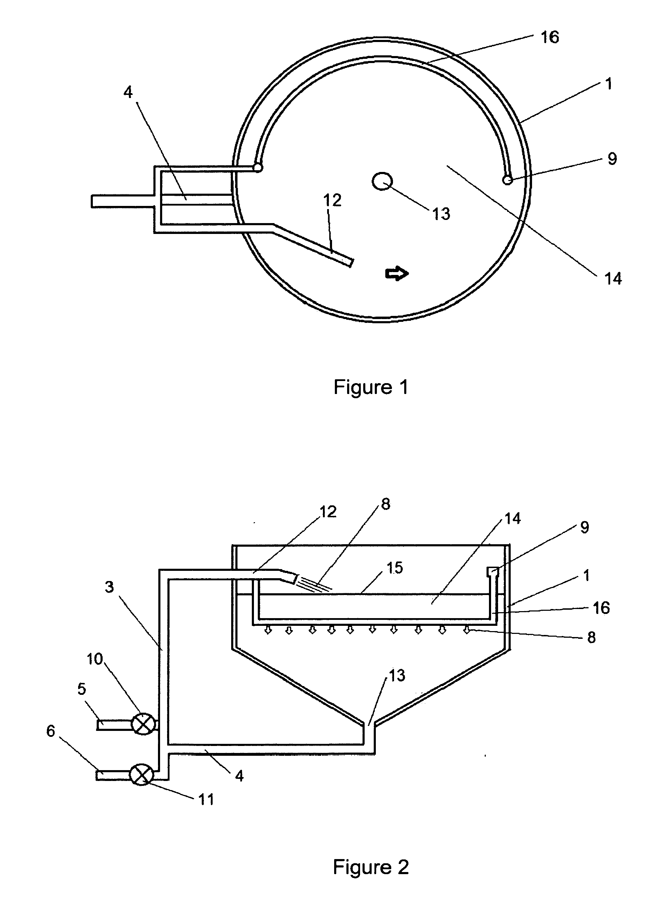

Figure 1 shows a plan view of the pond without the lid and its elements.
Figure 2 shows a side sectional view of the pond without the lid and its elements.
Figure 3 shows a plan view of the pond with its hexagonal transparent lid.
Figure 4 shows a side view of the pond with its hexagonal transparent lid.

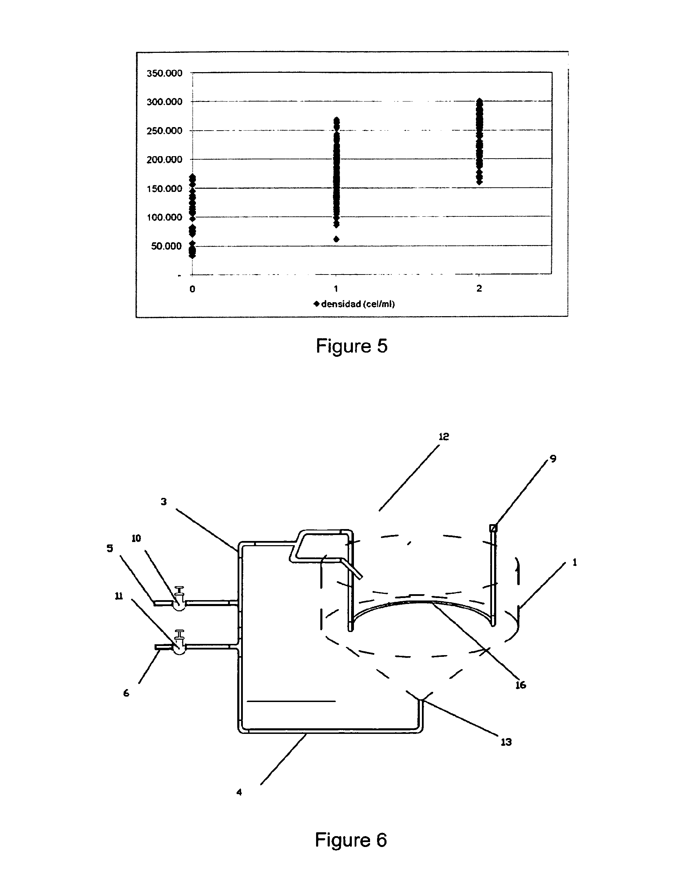

Figure 5 shows a graph of cells/ml. considering examples 1, 2 and 3
Figure 6 shows an isometric view of the installation
Figure 7 shows an installation and distribution with 6 groups or cells of three ponds each.

### DETAILED DESCRIPTION OF THE INVENTION

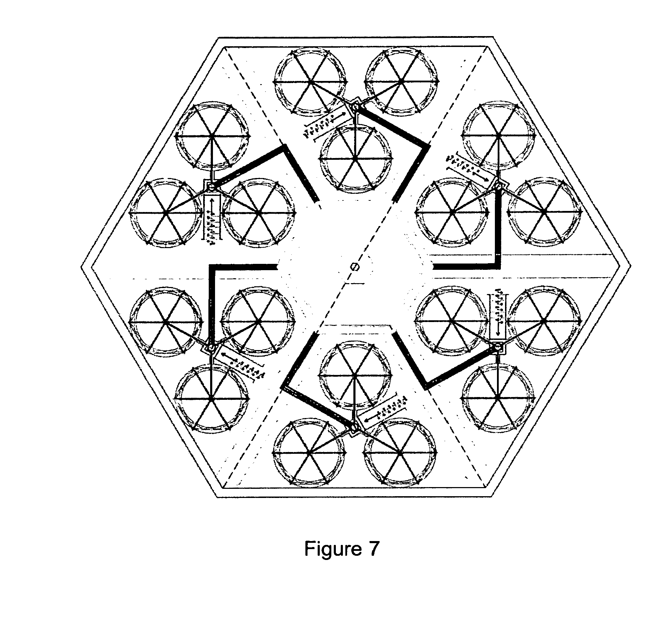

The culture system is designed considering escalation, that is, a spatial distribution of ponds that can easily increase the area of production by the incorporation of new cells (groups of three ponds connected to a central pumping system) (Figure 7).

Ponds are circular with a cylindrical mantle and conic bottom, preferably of fiberglass or coated cement. The preferred form of construction of the pond is that where the pond diameter is greater than its height.

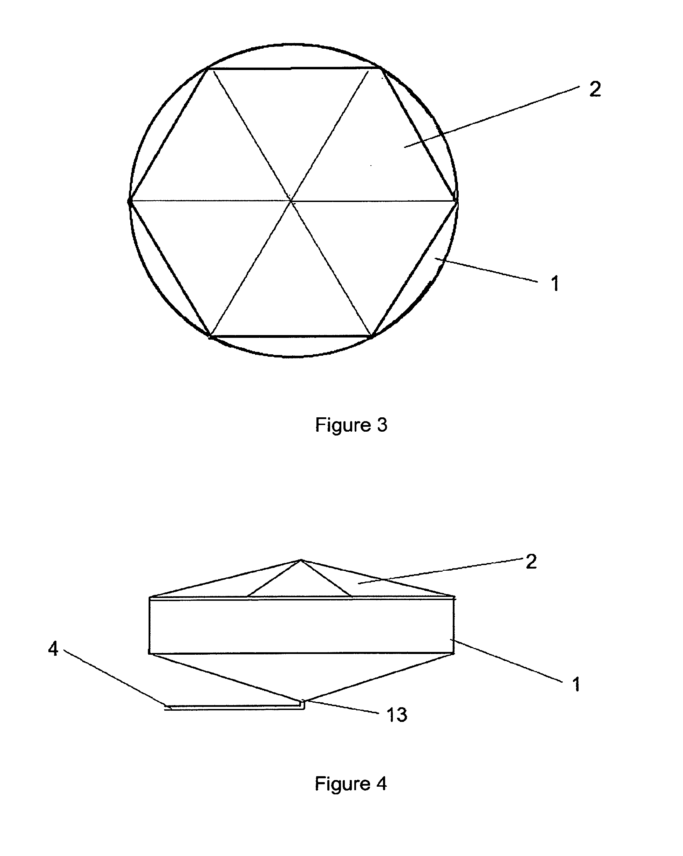

During operation, ponds are covered by a hexagonal removable lid of a transparent material, preferably alveolar polycarbonate (Figures 3 and 4). For visual inspection of the pond interior, one of the faces of the hexagon can be removed.

The system keeps a stable temperature of the culture medium within a suitable range for the growth of microalgae. This is achieved because ponds are partially buried in the ground (Figure 4, dotted line), which allows to counteract daynight thermal oscillations and not suffer significant variations in the average temperature between winter and summer. The ideal range of pH for the operation of the system is between 6.0 and 11.0.

The culture system performs propulsion by air. This propulsion system operates connected to a blower outside the installation (5), this blower allows to feed several modular groups or cells at the same time, producing a mild pumping while performing the gas exchange between the culture medium and the air pumped. The culture medium is distributed into the ponds in the movement of recirculation to maintain a constant stirring, to this end each pond has in its interior a first aerator (12) which carries the culture medium and the air, consisting of a straight tube near the liquid surface and above it at an angle between 30° and 60° respect to the liquid surface, which discharges into the pond tangentially against the cylindrical mantle of such pond, forming a circular flow or vortex which is also part of the propulsion system of the culture medium inside the pond.

The second PVC aerator carries the culture medium and the air, and consists of a semicircular tube (16) with a plug at one end (9), located near the bottom of such pond and which is part of the propulsion system of the culture medium inside the pond. Said second aerator comprises perforations through which the mixture of air or gas and culture medium is injected into the pond, such perforations are directed towards the bottom of the pond in order to prevent clogging of the holes by decantation of the cells in the culture (8).

Loading of the ponds with the culture medium and algae is made through the hydraulic line (6). Once the pond (1) is filled with culture medium (14), recirculation starts. Passage of pressurized gasses (5) is opened through the gas inlet valve (10), and these may be air, carbon dioxide or a mixture thereof, and the hydraulic line (11) is closed. The gases push the culture medium upwards and partially mix with water, which returns to the pond (1) entering tangentially against the circle. This movement generates a circular current. The culture medium is mixed and releases photosynthesis gases that mix with the pressurized gases at the top of the pond.

After spinning around the system, the culture medium returns to the center of the pond to the recirculation line (4). This line allows filling of the ponds with culture medium (14), incorporation of the inoculums, and harvesting is performed.

Air line (5) incorporates gases into the system (air) to supply power for la recirculation.

The system described in this application has no moving or metallic parts, which gives it a great versatility with respect to the photoreactors described in the state of the art. This feature makes it possible to use the invention for culturing microalgae either from fresh or salt water, among which are *Arthrospira platensis, Monoraphidium graphitti, Chlorella vulgaris, Anabaena variabilis* and *Nannochloropsis oculata, Chlorella neustonica,* respectively.

### EXAMPLES

### Example 1:

Growth of the *Arthrospira platensis* microalgae in the photobioreactor described in this application:
- Modified Zarrouk culture medium is used, in an agricultural degree;
- Ambient air is used as a source of CO₂;
- Density measurements were performed by cell counting using Sedgewick-Rafter chamber. The results are expressed in cells per milliliter.
- Harvest was weighed completely dry and is expressed in grams. Performance calculations are made by adding the period harvesting, divided by the culture area (14 m²) and the number of days in the period, the results being expressed in grams per square meter per day (g/m²/day). Rest days and no-harvesting days are included.

### Example 2:

Under standard culture conditions for *Arthrospira platensis* described in example 1, a continuous harvesting was conducted for 1 week. As a harvesting criterion, harvesting was performed each time the system exceeded an average of 180,000 cells per milliliter.

**Table 1:**

| Harvesting week 1 | | | | | | |
|---|---|---|---|---|---|---|
| **Date** | **Harvest Volume¹** | **Harvesting Time²** | **Dry Weight (Net)³** | **Harvest Volume⁴** | **Harvesting %⁵** | **Accumulated ⁶** |
| 2/7/2011 | 13 | 33 | 209 | 429 | 8% | 209.0 |
| 2/8/2011 | 14.3 | 30 | 215 | 429 | 8% | 424.0 |
| 2/10/2011 | 14.8 | 60 | 296 | 888 | 17% | 720.0 |
| 2/11/2011 | 13 | 90 | 327 | 1170 | 22% | **1047.0** |
| Total time | 5 days | | Average Productivity | | **15** g/m²/day | |
| Total Productivity | 1047.0 g | | Harvested Quantity | | 56% | |

| | | | | | | |
|---|---|---|---|---|---|---|
| ¹: expressed in l/min; ²: in minutes; ³: in grams; ⁴: l * min; ⁵: % of total volume 5.200 l; ⁶: grams harvested. | | | | | | |

### Example 2:

Under standard culture conditions for *Arthrospira platensis,* a continuous harvesting was conducted for a 1 week. This time the secondary bubbler was incorporated, which aims to increase turbulence, improve incorporation of CO₂ into the system and degassing of the medium. As a harvesting criterion, harvesting was performed each time the system exceeded an average of 180,000 cells per milliliter. The following table shows the harvesting results.

**Table 2:**

| Harvesting week 2 | | | | | | |
|---|---|---|---|---|---|---|
| **Date** | **Harvest Volume¹** | **Harvesting Time²** | **Dry Weight (Net)³** | **Harvest Volume⁴** | **Harvesting %⁵** | **Accumulated ⁶** |
| 3/1/2011 | 21 | 30 | 214 | 630 | 12% | 214.0 |
| 3/2/2011 | 13 | 30 | 184 | 390 | 7% | 398.0 |
| 3/3/2011 | 10 | 60 | 344 | 600 | 12% | 742.0 |
| 3/4/2011 | 12 | 60 | 279 | 720 | 13% | 1021.0 |
| 3/5/2011 | 18 | 120 | 557 | 2160 | 41% | **1578.0** |
| Total time | 5 days | | Average Productivity | | | **22.5** g/m²/day |
| Total Productivity | 1578.0 g | | Harvested Quantity | | 86% | |

| | | | | | | |
|---|---|---|---|---|---|---|
| ¹: expressed in I/min; ²: in minutes; ³: in grams; ⁴: l * min; ⁵: % of total volume 5.200 l; ⁶: grams harvested. | | | | | | |

The following table shows the difference in performance after incorporating the secondary bubbler.

**Table 3: Comparison between periods without the tube and those with the tube.**

| **Time** | **Average cells/ml** | **Increase (%)** | **Average performance (g/m²/day)** | **Increase (%)** |
|---|---|---|---|---|
| 0 | 89,280 | | | |
| 1 | 176,503 | | 5.1 | |
| 2 | 244,328 | 38% | 8.1 | 57% |

### REFERENCES

Murray Moo-Young and Yusuf Chisti (1994). Bioreactor applications in waste treatment. Resources, Conservation and Recycling. Vol 11, 13-24
Asterio Sánchez Miróna, M. Carmen Cerón Garcia, Antonio Contreras Gómeza, Francisco Garcia Camachoa, Emilio Molina Grimaa and Yusuf Chisti (2003). Shear stress tolerance and biochemical characterization of Phaeodactylum tricornutum in quasi steady-state continuous culture in outdoor photobioreactors. Biochemical Engineering Journal. Vol 16(3), 287-297
Han Xua, Xiaoling Miao and Qingyu Wu (2006). High quality biodiesel production from a microalga Chlorella protothecoides by heterotrophic growth in fermenters. Journal of Biotechnology. Vol 126(4), 499-507
Shakeel A. Khan, Rashmib, Mir Z. Hussaina, S. Prasada and U.C. Banerjeeb (2009). Prospects of biodiesel production from microalgae in India. Renewable and Sustainable Energy Reviews, Vol 13(9), 2361-2372.
Raja, R., Hemaiswarya, S., Kumar, N. A., Sridhar, S., & Rengasamy, R. (2008). A perspective on the biotechnological potential of microalgae. Critical Reviews in Microbiology, 34(2), 77-88.

## Claims

1. A closed microalgae culture system that provides a greater control of the culture and the possibility to incorporate gases into the culture medium, wherein:
a) The system uses a cellular model comprising groups of three bioreactor-type culture units, distributed so as to facilitate its escalation.
b) Each culture unit of such cellular model comprises:
- A pond with a cylindrical mantle and conic bottom buried in the ground
- A hexagonal transparent lid, preferably made of polycarbonate, installed on such pond.
- A first aerator which carries the culture medium and the air, consisting of a straight tube near the liquid surface and above it at an angle between 30° and 60° respect to the liquid surface, which discharges into the pond tangentially against the cylindrical mantle of such pond, forming a circular flow or vortex which is also part of the propulsion system of the culture medium inside the pond;
- A second PVC aerator which carries the culture medium and the air consisting of a semicircular tube with a plug at one end located near the bottom of such pond and which is part of the propulsion system of the culture medium inside the pond, said second aerator comprises perforations through which the mixture of air or gas and culture medium is injected into the pond, such perforations are directed towards the bottom of the pond.
- A recirculation line from the bottom of the pond to the semicircular and tangential aerators, where an air injection line is inserted.
- A pipe and a valve for gas inlet
- A pipe and a valve for liquid inlet

2. The microalgae culture system according to claim 1, wherein the injection of air into the system is done by using an external blowing system which can feed several cellular model units, such blower injects a pressure preferably of 0.1 bar

3. The microalgae culture system according to claim 1, wherein the gas inlet line also allows the direct injection of carbon dioxide.

4. The microalgae culture system according to claim 1, wherein the combination of flows from the semicircular aerator and the straight aerator is used to improve aeration.

5. The microalgae culture system according to claim 1, wherein the flow of the perforations of the second aerator oriented towards the bottom of the pond is also used to prevent decantation in such pond.

6. The microalgae culture system according to claim 1, wherein the pond is buried in the ground for improvements in thermal isolation and to keep a constant temperature of the culture medium.

7. The microalgae culture system according to claim 1, wherein the inoculums are loaded directly into the pond.

8. The microalgae culture system according to claim 1, wherein the pH used in the culture medium is preferably between 6.0 and 11.0.

9. The microalgae culture system according to claim 1, wherein the system can be used either with fresh or salt water since it has no metallic parts.

10. The microalgae culture system according to claim 1, wherein the preferred form of construction of the pond is that where the pond diameter is greater than its height.

11. Method for using a culture system, wherein the method consists in:
a) Provide a culture unit comprising:
- A pond with a cylindrical mantle and conic bottom
- A hexagonal transparent lid, preferably made of polycarbonate, installed on such pond.
- A first aerator which carries the culture medium and the air, consisting of a straight tube near the liquid surface and above it at an angle between 30° and 60° respect to the liquid surface, which discharges into the pond tangentially against the cylindrical mantle of such pond, forming a circular flow or vortex which is also part of the propulsion system of the culture medium inside the pond and works in combination with the first aerator.
- A second PVC aerator which carries the culture medium and the air consisting of a semicircular tube with a plug at one end located near the bottom of such pond and which is part of the propulsion system of the culture medium inside the pond, said second aerator comprises perforations through which the mixture of air or gas and culture medium is injected into the pond, such perforations are directed towards the bottom of the pond.
- A recirculation line from the bottom of the pond to the semicircular and tangential aerators, where an air injection line is inserted.
- A pipe and a valve for gas inlet
- A pipe and a valve for liquid inlet
b) A pipe for liquid inlet, through which the pond is loaded with culture medium and microalgae.
c) Once the pond is filled, recirculation starts, and the liquid inlet valve is closed and the gas inlet valve is opened
d) Open the gas inlet valve to allow gases to push the culture medium upwards and partially mix with water, which returns to the pond entering through primary and secondary aerators.
e) Allow that, after spinning around the system, the culture medium returns to the center of the pond to the recirculation line.
f) Once the relevant measurements are performed, harvest the medium using the recirculation line.
